# EUROPEAN PATENT APPLICATION

(11) **EP 4 036 237 A1**
(43) Date of publication of application: **03.08.2022**
(21) Application number: 22153626.1
(22) Date of filing: 27.01.2022
(51) Int. Cl.: C12N 15/66, C07K 14/195, C12N 9/12, C12N 15/62, C12N 15/70, C12Q 1/6844

(54) **PWO-NEQSSB POLYMERASE, METHOD OF ITS PREPARATION, RECOMBINANT PLASMID, PRIMERS AND THE USE OF POLYMERASE**

(30) Priority: 27.01.2021 PL 43678421
(71) Applicant: Instytut Biotechnologii I Medycyny Molekularnej, 80-180 Gdansk (PL)
(72) Inventor: SKWARECKA, Marta, 87-820 Kowal (PL); SZEMIAKO, Kasjan, 80-287 Gdansk (PL); OLSZEWSKI, Marcin, 80-283 Gdansk (PL); ZOLEDOWSKA, Sabina, 80-423 Gdansk (PL); NIDZWORSKI, Dawid, 80-180 Gdansk (PL)
(74) Representative: Matyka, Malgorzata

(57) **Abstract**

The subject of the invention is Pwo-NeqSSB polymerase and the method of its cloning. In addition, the subject of the invention is an isolated recombinant plasmid, primers and the use of polymerase to replication specific sequences of the SARS CoV-2 virus.

SEQUENCE LISTING

<110> Instytut Biotechnologii i Medycyny Molekularnej

<120> Polimeraza Pwo-NeqSSB, sposób jej otrzymywania, plazmid
rekombinantowy, startery oraz zastosowanie polimerazy.

<130> 1EP/MM/2022

<150> PLP.436784
<151> 2021-01-27

<160> 9

<170> BiSSAP 1.3.6

<210> 1
<211> 1023
<212> PRT
<213> Artificial Sequence

<220>
<223> fusion 2 proteins

<400> 1
Met Ile Leu Asp Val Asp Tyr Ile Thr Glu Glu Gly Lys Pro Val Ile
1               5                   10                  15
Arg Leu Phe Lys Lys Glu Asn Gly Lys Phe Lys Ile Glu His Asp Arg
            20                  25                  30
Thr Phe Arg Pro Tyr Ile Tyr Ala Leu Leu Arg Asp Asp Ser Lys Ile
        35                  40                  45
Glu Glu Val Lys Lys Ile Thr Gly Glu Arg His Gly Lys Ile Val Arg
    50                  55                  60
Ile Val Asp Val Glu Lys Val Glu Lys Lys Phe Leu Gly Lys Pro Ile
65                  70                  75                  80
Thr Val Trp Lys Leu Tyr Leu Glu His Pro Gln Asp Val Pro Thr Ile
                85                  90                  95
Arg Glu Lys Val Arg Glu His Pro Ala Val Val Asp Ile Phe Glu Tyr
            100                 105                 110
Asp Ile Pro Phe Ala Lys Arg Tyr Leu Ile Asp Lys Gly Leu Ile Pro
        115                 120                 125
Met Glu Gly Glu Glu Glu Leu Lys Ile Leu Ala Phe Asp Ile Glu Thr
    130                 135                 140
Leu Tyr His Glu Gly Glu Glu Phe Gly Lys Gly Pro Ile Ile Met Ile
145                 150                 155                 160
Ser Tyr Ala Asp Glu Asn Glu Ala Lys Val Ile Thr Trp Lys Asn Ile
                165                 170                 175
Asp Leu Pro Tyr Val Glu Val Val Ser Ser Glu Arg Glu Met Ile Lys
            180                 185                 190
Arg Phe Leu Arg Ile Ile Arg Glu Lys Asp Pro Asp Ile Ile Val Thr
        195                 200                 205
Tyr Asn Gly Asp Ser Phe Asp Phe Pro Tyr Leu Ala Lys Arg Ala Glu
    210                 215                 220
Lys Leu Gly Ile Lys Leu Thr Ile Gly Arg Asp Gly Ser Glu Pro Lys
225                 230                 235                 240
Met Gln Arg Ile Gly Asp Met Thr Ala Val Glu Val Lys Gly Arg Ile
                245                 250                 255
His Phe Asp Leu Tyr His Val Ile Thr Arg Thr Ile Asn Leu Pro Thr
            260                 265                 270
Tyr Thr Leu Glu Ala Val Tyr Glu Ala Ile Phe Gly Lys Pro Lys Glu
        275                 280                 285
Lys Val Tyr Ala Asp Glu Ile Ala Lys Ala Trp Glu Ser Gly Glu Asn

290                 295                 300
Leu Glu Arg Val Ala Lys Tyr Ser Met Glu Asp Ala Lys Ala Thr Tyr
305                 310                 315                 320
Glu Leu Gly Lys Glu Phe Leu Pro Met Glu Ile Gln Leu Ser Arg Leu
                325                 330                 335
Val Gly Gln Pro Leu Trp Asp Val Ser Arg Ser Ser Thr Gly Asn Leu
            340                 345                 350
Val Glu Trp Phe Leu Leu Arg Lys Ala Tyr Glu Arg Asn Glu Val Ala
        355                 360                 365
Pro Asn Lys Pro Ser Glu Glu Glu Tyr Gln Arg Arg Leu Arg Glu Ser
    370                 375                 380
Tyr Thr Gly Gly Phe Val Lys Glu Pro Glu Lys Gly Leu Trp Glu Asn
385                 390                 395                 400
Ile Val Tyr Leu Asp Phe Arg Ala Leu Tyr Pro Ser Ile Ile Ile Thr
                405                 410                 415
His Asn Val Ser Pro Asp Thr Leu Asn Leu Glu Gly Cys Lys Asn Tyr
            420                 425                 430
Asp Ile Ala Pro Gln Val Gly His Lys Phe Cys Lys Asp Ile Pro Gly
        435                 440                 445
Phe Ile Pro Ser Leu Leu Gly His Leu Leu Glu Glu Arg Gln Lys Ile
    450                 455                 460
Lys Thr Lys Met Lys Glu Thr Gln Asp Pro Ile Glu Lys Ile Leu Leu
465                 470                 475                 480
Asp Tyr Arg Gln Lys Ala Ile Lys Leu Leu Ala Asn Ser Phe Tyr Gly
                485                 490                 495
Tyr Tyr Gly Tyr Ala Lys Ala Arg Trp Tyr Cys Lys Glu Cys Ala Glu
            500                 505                 510
Ser Val Thr Ala Trp Gly Arg Lys Tyr Ile Glu Leu Val Trp Lys Glu
        515                 520                 525
Leu Glu Glu Lys Phe Gly Phe Lys Val Leu Tyr Ile Asp Thr Asp Gly
    530                 535                 540
Leu Tyr Ala Thr Ile Pro Gly Gly Glu Ser Glu Glu Ile Lys Lys Lys
545                 550                 555                 560
Ala Leu Glu Phe Val Lys Tyr Ile Asn Ser Lys Leu Pro Gly Leu Leu
                565                 570                 575
Glu Leu Glu Tyr Glu Gly Phe Tyr Lys Arg Gly Phe Phe Val Thr Lys
            580                 585                 590
Lys Arg Tyr Ala Val Ile Asp Glu Glu Gly Lys Val Ile Thr Arg Gly
        595                 600                 605
Leu Glu Ile Val Arg Arg Asp Trp Ser Glu Ile Ala Lys Glu Thr Gln
    610                 615                 620
Ala Arg Val Leu Glu Thr Ile Leu Lys His Gly Asp Val Glu Glu Ala
625                 630                 635                 640
Val Arg Ile Val Lys Glu Val Ile Gln Lys Leu Ala Asn Tyr Glu Ile
                645                 650                 655
Pro Pro Glu Lys Leu Ala Ile Tyr Glu Gln Ile Thr Arg Pro Leu His
            660                 665                 670
Glu Tyr Lys Ala Ile Gly Pro His Val Ala Val Ala Lys Lys Leu Ala
        675                 680                 685
Ala Lys Gly Val Lys Ile Lys Pro Gly Met Val Ile Gly Tyr Ile Val
    690                 695                 700
Leu Arg Gly Asp Gly Pro Ile Ser Asn Arg Ala Ile Leu Ala Glu Glu
705                 710                 715                 720
Tyr Asp Pro Lys Lys His Lys Tyr Asp Ala Glu Tyr Tyr Ile Glu Asn
                725                 730                 735
Gln Val Leu Pro Ala Val Leu Arg Ile Leu Glu Gly Phe Gly Tyr Arg
            740                 745                 750
Lys Glu Asp Leu Arg Tyr Gln Lys Thr Arg Gln Val Gly Leu Thr Ser
        755                 760                 765
Trp Leu Asn Ile Lys Lys Ser Gly Ser Gly Gly Val Asp Asp Glu Glu
    770                 775                 780
Glu Leu Ile Gln Leu Ile Ile Glu Lys Thr Gly Lys Ser Arg Glu Glu
785                 790                 795                 800

Ile Glu Lys Met Val Glu Glu Lys Ile Lys Ala Phe Asn Asn Leu Ile
                805                 810                 815
Ser Arg Arg Gly Ala Leu Leu Leu Val Ala Lys Lys Leu Gly Val Leu
            820                 825                 830
Tyr Lys Asn Thr Pro Lys Glu Lys Lys Ile Gly Glu Leu Glu Ser Trp
        835                 840                 845
Glu Tyr Val Lys Val Lys Gly Lys Ile Leu Lys Ser Phe Gly Leu Ile
    850                 855                 860
Ser Tyr Ser Lys Gly Lys Phe Gln Pro Ile Ile Leu Gly Asp Glu Thr
865                 870                 875                 880
Gly Thr Ile Lys Ala Ile Ile Trp Asn Thr Asp Lys Glu Leu Pro Glu
                885                 890                 895
Asn Thr Val Ile Glu Ala Ile Gly Lys Thr Lys Ile Asn Lys Lys Thr
            900                 905                 910
Gly Asn Leu Glu Leu His Ile Asp Ser Tyr Lys Ile Leu Glu Ser Asp
        915                 920                 925
Leu Glu Ile Lys Pro Gln Lys Gln Glu Phe Val Gly Ile Cys Ile Val
    930                 935                 940
Lys Tyr Pro Lys Lys Gln Thr Gln Lys Gly Thr Ile Val Ser Lys Ala
945                 950                 955                 960
Ile Leu Thr Ser Leu Asp Arg Glu Leu Pro Val Val Tyr Phe Asn Asp
                965                 970                 975
Phe Asp Trp Glu Ile Gly His Ile Tyr Lys Val Tyr Gly Lys Leu Lys
            980                 985                 990
Lys Asn Ile Lys Thr Gly Lys Ile Glu Phe Phe Ala Asp Lys Val Glu
        995                 1000                1005
Glu Ala Thr Leu Lys Asp Leu Lys Ala Phe Lys Gly Glu Ala Asp
    1010                1015                1020

<210> 2
<211> 775
<212> PRT
<213> Pyrococcus furiosus

<400> 2
Met Ile Leu Asp Val Asp Tyr Ile Thr Glu Glu Gly Lys Pro Val Ile
1               5                   10                  15
Arg Leu Phe Lys Lys Glu Asn Gly Lys Phe Lys Ile Glu His Asp Arg
            20                  25                  30
Thr Phe Arg Pro Tyr Ile Tyr Ala Leu Leu Arg Asp Asp Ser Lys Ile
        35                  40                  45
Glu Glu Val Lys Lys Ile Thr Gly Glu Arg His Gly Lys Ile Val Arg
    50                  55                  60
Ile Val Asp Val Glu Lys Val Glu Lys Lys Phe Leu Gly Lys Pro Ile
65                  70                  75                  80
Thr Val Trp Lys Leu Tyr Leu Glu His Pro Gln Asp Val Pro Thr Ile
                85                  90                  95
Arg Glu Lys Val Arg Glu His Pro Ala Val Val Asp Ile Phe Glu Tyr
            100                 105                 110
Asp Ile Pro Phe Ala Lys Arg Tyr Leu Ile Asp Lys Gly Leu Ile Pro
        115                 120                 125
Met Glu Gly Glu Glu Glu Leu Lys Ile Leu Ala Phe Asp Ile Glu Thr
    130                 135                 140
Leu Tyr His Glu Gly Glu Glu Phe Gly Lys Gly Pro Ile Ile Met Ile
145                 150                 155                 160
Ser Tyr Ala Asp Glu Asn Glu Ala Lys Val Ile Thr Trp Lys Asn Ile
                165                 170                 175
Asp Leu Pro Tyr Val Glu Val Val Ser Ser Glu Arg Glu Met Ile Lys
            180                 185                 190
Arg Phe Leu Arg Ile Ile Arg Glu Lys Asp Pro Asp Ile Ile Val Thr
        195                 200                 205
Tyr Asn Gly Asp Ser Phe Asp Phe Pro Tyr Leu Ala Lys Arg Ala Glu

210                 215                 220
Lys Leu Gly Ile Lys Leu Thr Ile Gly Arg Asp Gly Ser Glu Pro Lys
225                 230                 235                 240
Met Gln Arg Ile Gly Asp Met Thr Ala Val Glu Val Lys Gly Arg Ile
                245                 250                 255
His Phe Asp Leu Tyr His Val Ile Thr Arg Thr Ile Asn Leu Pro Thr
            260                 265                 270
Tyr Thr Leu Glu Ala Val Tyr Glu Ala Ile Phe Gly Lys Pro Lys Glu
        275                 280                 285
Lys Val Tyr Ala Asp Glu Ile Ala Lys Ala Trp Glu Ser Gly Glu Asn
    290                 295                 300
Leu Glu Arg Val Ala Lys Tyr Ser Met Glu Asp Ala Lys Ala Thr Tyr
305                 310                 315                 320
Glu Leu Gly Lys Glu Phe Leu Pro Met Glu Ile Gln Leu Ser Arg Leu
                325                 330                 335
Val Gly Gln Pro Leu Trp Asp Val Ser Arg Ser Ser Thr Gly Asn Leu
            340                 345                 350
Val Glu Trp Phe Leu Leu Arg Lys Ala Tyr Glu Arg Asn Glu Val Ala
        355                 360                 365
Pro Asn Lys Pro Ser Glu Glu Glu Tyr Gln Arg Arg Leu Arg Glu Ser
    370                 375                 380
Tyr Thr Gly Gly Phe Val Lys Glu Pro Glu Lys Gly Leu Trp Glu Asn
385                 390                 395                 400
Ile Val Tyr Leu Asp Phe Arg Ala Leu Tyr Pro Ser Ile Ile Ile Thr
                405                 410                 415
His Asn Val Ser Pro Asp Thr Leu Asn Leu Glu Gly Cys Lys Asn Tyr
            420                 425                 430
Asp Ile Ala Pro Gln Val Gly His Lys Phe Cys Lys Asp Ile Pro Gly
        435                 440                 445
Phe Ile Pro Ser Leu Leu Gly His Leu Leu Glu Glu Arg Gln Lys Ile
    450                 455                 460
Lys Thr Lys Met Lys Glu Thr Gln Asp Pro Ile Glu Lys Ile Leu Leu
465                 470                 475                 480
Asp Tyr Arg Gln Lys Ala Ile Lys Leu Leu Ala Asn Ser Phe Tyr Gly
                485                 490                 495
Tyr Tyr Gly Tyr Ala Lys Ala Arg Trp Tyr Cys Lys Glu Cys Ala Glu
            500                 505                 510
Ser Val Thr Ala Trp Gly Arg Lys Tyr Ile Glu Leu Val Trp Lys Glu
        515                 520                 525
Leu Glu Glu Lys Phe Gly Phe Lys Val Leu Tyr Ile Asp Thr Asp Gly
    530                 535                 540
Leu Tyr Ala Thr Ile Pro Gly Gly Glu Ser Glu Glu Ile Lys Lys Lys
545                 550                 555                 560
Ala Leu Glu Phe Val Lys Tyr Ile Asn Ser Lys Leu Pro Gly Leu Leu
                565                 570                 575
Glu Leu Glu Tyr Glu Gly Phe Tyr Lys Arg Gly Phe Phe Val Thr Lys
            580                 585                 590
Lys Arg Tyr Ala Val Ile Asp Glu Glu Gly Lys Val Ile Thr Arg Gly
        595                 600                 605
Leu Glu Ile Val Arg Arg Asp Trp Ser Glu Ile Ala Lys Glu Thr Gln
    610                 615                 620
Ala Arg Val Leu Glu Thr Ile Leu Lys His Gly Asp Val Glu Glu Ala
625                 630                 635                 640
Val Arg Ile Val Lys Glu Val Ile Gln Lys Leu Ala Asn Tyr Glu Ile
                645                 650                 655
Pro Pro Glu Lys Leu Ala Ile Tyr Glu Gln Ile Thr Arg Pro Leu His
            660                 665                 670
Glu Tyr Lys Ala Ile Gly Pro His Val Ala Val Ala Lys Lys Leu Ala
        675                 680                 685
Ala Lys Gly Val Lys Ile Lys Pro Gly Met Val Ile Gly Tyr Ile Val
    690                 695                 700
Leu Arg Gly Asp Gly Pro Ile Ser Asn Arg Ala Ile Leu Ala Glu Glu
705                 710                 715                 720

Tyr Asp Pro Lys Lys His Lys Tyr Asp Ala Glu Tyr Tyr Ile Glu Asn
                725                 730                 735
Gln Val Leu Pro Ala Val Leu Arg Ile Leu Glu Gly Phe Gly Tyr Arg
            740                 745                 750
Lys Glu Asp Leu Arg Tyr Gln Lys Thr Arg Gln Val Gly Leu Thr Ser
        755                 760                 765
Trp Leu Asn Ile Lys Lys Ser
    770                 775

<210> 3
<211> 6
<212> PRT
<213> Artificial Sequence

<220>
<223> Linker

<400> 3
Gly Ser Gly Gly Val Asp
1               5

<210> 4
<211> 242
<212> PRT
<213> Bacteria <prokaryote>

<400> 4
Asp Glu Glu Glu Leu Ile Gln Leu Ile Ile Glu Lys Thr Gly Lys Ser
1               5                   10                  15
Arg Glu Glu Ile Glu Lys Met Val Glu Glu Lys Ile Lys Ala Phe Asn
            20                  25                  30
Asn Leu Ile Ser Arg Arg Gly Ala Leu Leu Leu Val Ala Lys Lys Leu
        35                  40                  45
Gly Val Leu Tyr Lys Asn Thr Pro Lys Glu Lys Lys Ile Gly Glu Leu
    50                  55                  60
Glu Ser Trp Glu Tyr Val Lys Val Lys Gly Lys Ile Leu Lys Ser Phe
65                  70                  75                  80
Gly Leu Ile Ser Tyr Ser Lys Gly Lys Phe Gln Pro Ile Ile Leu Gly
                85                  90                  95
Asp Glu Thr Gly Thr Ile Lys Ala Ile Ile Trp Asn Thr Asp Lys Glu
            100                 105                 110
Leu Pro Glu Asn Thr Val Ile Glu Ala Ile Gly Lys Thr Lys Ile Asn
        115                 120                 125
Lys Lys Thr Gly Asn Leu Glu Leu His Ile Asp Ser Tyr Lys Ile Leu
    130                 135                 140
Glu Ser Asp Leu Glu Ile Lys Pro Gln Lys Gln Glu Phe Val Gly Ile
145                 150                 155                 160
Cys Ile Val Lys Tyr Pro Lys Lys Gln Thr Gln Lys Gly Thr Ile Val
                165                 170                 175
Ser Lys Ala Ile Leu Thr Ser Leu Asp Arg Glu Leu Pro Val Val Tyr
            180                 185                 190
Phe Asn Asp Phe Asp Trp Glu Ile Gly His Ile Tyr Lys Val Tyr Gly
        195                 200                 205
Lys Leu Lys Lys Asn Ile Lys Thr Gly Lys Ile Glu Phe Phe Ala Asp
    210                 215                 220
Lys Val Glu Glu Ala Thr Leu Lys Asp Leu Lys Ala Phe Lys Gly Glu
225                 230                 235                 240
Ala Asp

<210> 5

<211> 8371
<212> DNA
<213> Artificial Sequence

<220>
<223> Recombinant plasmide pET30 EKLIC

<400> 5
tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg aattaattct tagaaaaact catcgagcat caaatgaaac tgcaatttat 600

tcatatcagg attatcaata ccatattttt gaaaaagccg tttctgtaat gaaggagaaa 660

actcaccgag gcagttccat aggatggcaa gatcctggta tcggtctgcg attccgactc 720

gtccaacatc aatacaacct attaatttcc cctcgtcaaa aataaggtta tcaagtgaga 780

aatcaccatg agtgacgact gaatccggtg agaatggcaa aagtttatgc atttctttcc 840

agacttgttc aacaggccag ccattacgct cgtcatcaaa atcactcgca tcaaccaaac 900

cgttattcat tcgtgattgc gcctgagcga gacgaaatac gcgatcgctg ttaaaaggac 960

aattacaaac aggaatcgaa tgcaaccggc gcaggaacac tgccagcgca tcaacaatat 1020

tttcacctga atcaggatat tcttctaata cctggaatgc tgttttcccg gggatcgcag 1080

tggtgagtaa ccatgcatca tcaggagtac ggataaaatg cttgatggtc ggaagaggca 1140

taaattccgt cagccagttt agtctgacca tctcatctgt aacatcattg gcaacgctac 1200

ctttgccatg tttcagaaac aactctggcg catcgggctt cccatacaat cgatagattg 1260

tcgcacctga ttgcccgaca ttatcgcgag cccatttata cccatataaa tcagcatcca 1320

tgttggaatt taatcgcggc ctagagcaag acgtttcccg ttgaatatgg ctcataacac 1380

cccttgtatt actgtttatg taagcagaca gttttattgt tcatgaccaa aatcccttaa 1440

cgtgagtttt cgttccactg agcgtcagac cccgtagaaa agatcaaagg atcttcttga 1500

gatccttttt ttctgcgcgt aatctgctgc ttgcaaacaa aaaaaccacc gctaccagcg 1560

gtggtttgtt tgccggatca agagctacca actctttttc cgaaggtaac tggcttcagc 1620

agagcgcaga taccaaatac tgtccttcta gtgtagccgt agttaggcca ccacttcaag 1680

aactctgtag caccgcctac atacctcgct ctgctaatcc tgttaccagt ggctgctgcc 1740

agtggcgata agtcgtgtct taccgggttg gactcaagac gatagttacc ggataaggcg 1800

cagcggtcgg gctgaacggg gggttcgtgc acacagccca gcttggagcg aacgacctac 1860

accgaactga gatacctaca gcgtgagcta tgagaaagcg ccacgcttcc cgaagggaga 1920

aaggcggaca ggtatccggt aagcggcagg gtcggaacag gagagcgcac gagggagctt 1980

ccagggggaa acgcctggta tctttatagt cctgtcgggt ttcgccacct ctgacttgag 2040

cgtcgatttt tgtgatgctc gtcagggggg cggagcctat ggaaaaacgc cagcaacgcg 2100

gcctttttac ggttcctggc cttttgctgg ccttttgctc acatgttctt tcctgcgtta 2160

tcccctgatt ctgtggataa ccgtattacc gcctttgagt gagctgatac cgctcgccgc 2220

agccgaacga ccgagcgcag cgagtcagtg agcgaggaag cggaagagcg cctgatgcgg 2280

tattttctcc ttacgcatct gtgcggtatt tcacaccgca tatatggtgc actctcagta 2340

caatctgctc tgatgccgca tagttaagcc agtatacact ccgctatcgc tacgtgactg 2400

ggtcatggct gcgccccgac acccgccaac acccgctgac gcgccctgac gggcttgtct 2460

gctcccggca tccgcttaca gacaagctgt gaccgtctcc gggagctgca tgtgtcagag 2520

gttttcaccg tcatcaccga aacgcgcgag gcagctgcgg taaagctcat cagcgtggtc 2580

gtgaagcgat tcacagatgt ctgcctgttc atccgcgtcc agctcgttga gtttctccag 2640

aagcgttaat gtctggcttc tgataaagcg ggccatgtta agggcggttt tttcctgttt 2700

ggtcactgat gcctccgtgt aagggggatt tctgttcatg ggggtaatga taccgatgaa 2760

acgagagagg atgctcacga tacgggttac tgatgatgaa catgcccggt tactggaacg 2820

ttgtgagggt aaacaactgg cggtatggat gcggcgggac cagagaaaaa tcactcaggg 2880

tcaatgccag cgcttcgtta atacagatgt aggtgttcca cagggtagcc agcagcatcc 2940

tgcgatgcag atccggaaca taatggtgca gggcgctgac ttccgcgttt ccagacttta 3000

cgaaacacgg aaaccgaaga ccattcatgt tgttgctcag gtcgcagacg ttttgcagca 3060

gcagtcgctt cacgttcgct cgcgtatcgg tgattcattc tgctaaccag taaggcaacc 3120

ccgccagcct agccgggtcc tcaacgacag gagcacgatc atgcgcaccc gtggggccgc 3180

catgccggcg ataatggcct gcttctcgcc gaaacgtttg gtggcgggac cagtgacgaa 3240

ggcttgagcg agggcgtgca agattccgaa taccgcaagc gacaggccga tcatcgtcgc 3300

gctccagcga aagcggtcct cgccgaaaat gacccagagc gctgccggca cctgtcctac 3360

gagttgcatg ataaagaaga cagtcataag tgcggcgacg atagtcatgc cccgcgccca 3420

ccggaaggag ctgactgggt tgaaggctct caagggcatc ggtcgagatc ccggtgccta 3480

atgagtgagc taacttacat taattgcgtt gcgctcactg cccgctttcc agtcgggaaa 3540

cctgtcgtgc cagctgcatt aatgaatcgg ccaacgcgcg gggagaggcg gtttgcgtat 3600

tgggcgccag ggtggttttt cttttcacca gtgagacggg caacagctga ttgcccttca 3660

ccgcctggcc ctgagagagt tgcagcaagc ggtccacgct ggtttgcccc agcaggcgaa 3720

aatcctgttt gatggtggtt aacggcggga tataacatga gctgtcttcg gtatcgtcgt 3780

atcccactac cgagatgtcc gcaccaacgc gcagcccgga ctcggtaatg gcgcgcattg 3840

cgcccagcgc catctgatcg ttggcaacca gcatcgcagt gggaacgatg ccctcattca 3900

gcatttgcat ggtttgttga aaaccggaca tggcactcca gtcgccttcc cgttccgcta 3960

tcggctgaat ttgattgcga gtgagatatt tatgccagcc agccagacgc agacgcgccg 4020

agacagaact taatgggccc gctaacagcg cgatttgctg gtgacccaat gcgaccagat 4080

gctccacgcc cagtcgcgta ccgtcttcat gggagaaaat aatactgttg atgggtgtct 4140

ggtcagagac atcaagaaat aacgccggaa cattagtgca ggcagcttcc acagcaatgg 4200

catcctggtc atccagcgga tagttaatga tcagcccact gacgcgttgc gcgagaagat 4260

tgtgcaccgc cgctttacag gcttcgacgc cgcttcgttc taccatcgac accaccacgc 4320

tggcacccag ttgatcggcg cgagatttaa tcgccgcgac aatttgcgac ggcgcgtgca 4380

gggccagact ggaggtggca acgccaatca gcaacgactg tttgcccgcc agttgttgtg 4440

ccacgcggtt gggaatgtaa ttcagctccg ccatcgccgc ttccactttt tcccgcgttt 4500

tcgcagaaac gtggctggcc tggttcacca cgcgggaaac ggtctgataa gagacaccgg 4560

catactctgc gacatcgtat aacgttactg gtttcacatt caccaccctg aattgactct 4620

cttccgggcg ctatcatgcc ataccgcgaa aggttttgcg ccattcgatg gtgtccggga 4680

tctcgacgct ctcccttatg cgactcctgc attaggaagc agcccagtag taggttgagg 4740

ccgttgagca ccgccgccgc aaggaatggt gcatgcaagg agatggcgcc caacagtccc 4800

ccggccacgg ggcctgccac catacccacg ccgaaacaag cgctcatgag cccgaagtgg 4860

cgagcccgat cttccccatc ggtgatgtcg gcgatatagg cgccagcaac cgcacctgtg 4920

gcgccggtga tgccggccac gatgcgtccg gcgtagagga tcgagatcga tctcgatccc 4980

gcgaaattaa tacgactcac tataggggaa ttgtgagcgg ataacaattc ccctctagaa 5040

ataattttgt ttaactttaa gaaggagata tacatatgat tttagatgtg gattacataa 5100

ctgaagaagg aaaacctgtt attaggctat tcaaaaaaga gaacggaaaa tttaagatag 5160

agcatgatag aacttttaga ccatacattt acgctcttct cagggatgat tcaaagattg 5220

aagaagttaa gaaaataacg ggggaaaggc atggaaagat tgtgagaatt gttgatgtag 5280

agaaggttga gaaaaagttt ctcggcaagc ctattaccgt gtggaaactt tatttggaac 5340

atccccaaga tgttcccact attagagaaa aagttagaga acatccagca gttgtggaca 5400

tcttcgaata cgatattcca tttgcaaaga gatacctcat cgacaaaggc ctaataccaa 5460

tggaggggga agaagagcta aagattcttg ccttcgatat agaaaccctc tatcacgaag 5520

gagaagagtt tggaaaaggc ccaattataa tgattagtta tgcagatgaa aatgaagcaa 5580

aggtgattac ttggaaaaac atagatcttc catacgttga ggttgtatca agcgagagag 5640

agatgataaa gagatttctc aggattatca gggagaagga tcctgacatt atagttactt 5700

ataatggaga ctcattcgac ttcccatatt tagcgaaaag ggcagaaaaa cttgggatta 5760

aattaaccat tggaagagat ggaagcgagc ccaagatgca gagaataggc gatatgacgg 5820

ctgtagaagt caagggaaga atacatttcg acttgtatca tgtaataaca aggacaataa 5880

atctcccaac atacacacta gaggctgtat atgaagcaat ttttggaaag ccaaaggaga 5940

aggtatacgc cgacgagata gcaaaagcct gggaaagtgg agagaacctt gagagagttg 6000

ccaaatactc gatggaagat gcaaaggcaa cttatgaact cgggaaagaa ttccttccaa 6060

tggaaattca gctttcaaga ttagttggac aacctttatg ggatgtttca aggtcaagca 6120

cagggaacct tgtagagtgg ttcttactta ggaaagccta cgaaagaaac gaagtagctc 6180

caaacaagcc aagtgaagag gagtatcaaa gaaggctcag ggagagctac acaggtggat 6240

tcgttaaaga gccagaaaag gggttgtggg aaaacatagt atacctagat tttagagccc 6300

tatatccctc gattataatt acccacaatg tttctcccga tactctaaat cttgagggat 6360

gcaagaacta tgatatcgct cctcaagtag gccacaagtt ctgcaaggac atccctggtt 6420

ttataccaag tctcttggga catttgttag aggaaagaca aaagattaag acaaaaatga 6480

aggaaactca agatcctata gaaaaaatac tccttgacta tagacaaaaa gcgataaaac 6540

tcttagcaaa ttctttctac ggatattatg gctatgcaaa agcaagatgg tactgtaagg 6600

agtgtgctga gagcgttact gcctggggaa gaaagtacat cgagttagta tggaaggagc 6660

tcgaagaaaa gtttggattt aaagtcctct acattgacac tgatggtctc tatgcaacta 6720

tcccaggagg agaaagtgag gaaataaaga aaaaggctct agaatttgta aaatacataa 6780

attcaaagct ccctggactg ctagagcttg aatatgaagg gttttataag aggggattct 6840

tcgttacgaa gaagaggtat gcagtaatag atgaagaagg aaaagtcatt actcgtggtt 6900

tagagatagt taggagagat tggagtgaaa ttgcaaaaga aactcaagct agagttttgg 6960

agacaatact aaaacacgga gatgttgaag aagctgtgag aatagtaaaa gaagtaatac 7020

aaaagcttgc caattatgaa attccaccag agaagctcgc aatatatgag cagataacaa 7080

gaccattaca tgagtataag gcgataggtc ctcacgtagc tgttgcaaag aaactagctg 7140

ctaaaggagt taaaataaag ccaggaatgg taattggata catagtactt agaggcgatg 7200

gtccaattag caatagggca attctagctg aggaatacga tcccaaaaag cacaagtatg 7260

acgcagaata ttacattgag aaccaggttc ttccagcggt acttaggata ttggagggat 7320

ttggatacag aaaggaagac ctcagatacc aaaagacaag acaagtcggc ctaacttcct 7380

ggcttaacat taaaaaatcc ggaagcggag gggtcgacga tgaagaggaa ctaatacaac 7440

taataataga aaaaactggc aaatctcgag aggaaataga aaaaatggtg gaagaaaaaa 7500

ttaaagcttt taacaattta atatctcgta ggggggcttt actattagta gcaaaaaaac 7560

ttggtgtttt gtataaaaac actccgaaag agaaaaaaat tggcgaatta gaaagctggg 7620

aatatgtaaa agtaaagggc aaaattctca aatcttttgg attaattagt tattcgaaag 7680

ggaaattcca acctattatt ttaggagacg aaaccggtac tattaaagct attatttgga 7740

ataccgataa agaattacct gaaaacactg taatagaagc tattgggaaa accaaaatta 7800

ataagaaaac tggcaattta gaattacata tagacagtta taaaatttta gaaagcgatt 7860

tagagataaa accccaaaag caagaatttg ttgggatttg catagttaaa tatccaaaaa 7920

aacaaaccca aaaaggcaca atagtatcga aagcaatttt aactagctta gatagggaat 7980

tgcctgtagt atatttcaac gattttgatt gggaaatagg ccatatatat aaagtatatg 8040

gaaagcttaa gaaaaacata aaaactggta aaatagaatt tttcgctgac aaagttgagg 8100

aagcaacatt aaaagatcta aaagctttta aaggagaggc cgattgacac caccaccacc 8160

accactaggg atccgaattc gagctccgtc gacaagcttg cggccgcact cgagcaccac 8220

caccaccacc actgagatcc ggctgctaac aaagcccgaa aggaagctga gttggctgct 8280

gccaccgctg agcaataact agcataaccc cttggggcct ctaaacgggt cttgaggggt 8340

tttttgctga aaggaggaac tatatccgga t 8371

<210> 6
<211> 57
<212> DNA
<213> Artificial Sequence

<220>
<223> clonic primer

<400> 6
aactttaaga aggagatata catatgattt tagatgtgga ttacataact gaagaag 57

<210> 7
<211> 49
<212> DNA
<213> Artificial Sequence

<220>
<223> clonic primer

<400> 7
tcgtcgaccc ctccgcttcc ggatttttta atgttaagcc aggaagtta 49

<210> 8
<211> 51
<212> DNA
<213> Artificial Sequence

<220>
<223> clonic primer

<400> 8
ccggaagcgg aggggtcgac gatgaagagg aactaataca actaataata g          51

<210> 9
<211> 78
<212> DNA
<213> Artificial Sequence

<220>
<223> clonic primer

<400> 9
gcaagcttgt cgacggagct cgaattcgga tccttagtgg tggtggtggt ggtgtcaatc     60

ggcctctcct ttaaaagc                                                   78

## Description

The subject of the invention is Pwo-NeqSSB polymerase and the method of its cloning. In addition, the subject of the invention is an isolated recombinant plasmid, primers and the use of polymerase to duplicate specific sequences of the SARS CoV-2 virus.

Thermophilic and hyperthermophilic organisms are a potential source of enzymes desired in various industries, due to their high stability at high temperatures. An example of proteins with wide application are thermostable DNA polymerases, whose optimal activity is achieved at temperatures above 70 ° C, and many of them retain their activity even above 100 ° C.

The structure of thermostable DNA polymerases differs from their counterparts of mesophilic origin. Their structure is stiffer and more packed. The stability of α-helix is ensured, by a reduced content of amino acid residues such as isoleucine or valine, among others, which significantly contribute to the occurrence of spherical stresses. In these enzymes, a significant increase in interactions was observed, such as: ionic, van der Waals or hydrophobic interactions, which also have a significant impact on the increase in protein thermo stability.

In molecular biology or genetic engineering techniques, thermostable DNA polymerases of bacterial origin are most often used: Thermus aquaticus, Thermus thermophilus, Thermotoga maritima, Bacillus stearothermophilus or archaea, i.e.: Thermococcus litoralis, Pyrococcus furiosus, Pyrococcus woesei. The main applications of these DNA polymerases are amplification of DNA fragments using PCR technique as well as DNA sequencing.

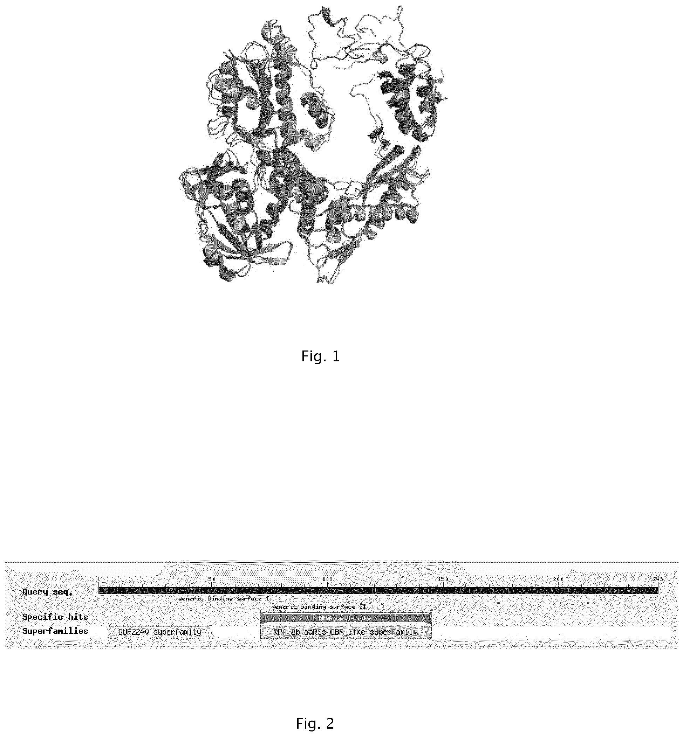

One of the best known DNA polymerases derived from Euryarcheot and used in biology and molecular diagnostics is Pwo DNA polymerase from Pyrococcus woesei. Its structure resembles a ring consisting of a 3'→5' exonuclease domain as well as a polymerization domain made up of a subdomain of thepalm, fingers and thumb (Figure 1).

The polymerization and correction activity of archaease DNA polymerases is associated with a loop in the domain responsible for exonucleolytic activity. It is a highly conservedelement and occurring only in this group of microorganisms. The amino acid residues that make up the loop interact with the positively charged edge of the thumb subdomain and allow the 3' ssDNA end to be bound to the centre of the active enzyme. Pwo DNA polymerase is characterized by high processivity and DNA replication fidelity, which is associated with the presence of the proofreading 3'→5' exonuclease domain. Its thermostability significantly exceeds that of bacterial DNA polymerase Taq, as its half-life is almost 3 h at 100°C. In addition, it shows increased processivity and is less likely to make errorsduring synthesis.

The NeqSSB protein belongs to the SSB (Single-Stranded DNA Binding protein) family. SSB proteins exhibit a variety of amino acid sequences and structure. Despite this, they all have a characteristic, strongly conserved, about 100 - amino acid oligonucleotide domain OB (Oligonucleotide/Oligosaccharide Binding Fold Domain). It is common in proteins with the ability to bind ssDNA, so it determines this basic and common feature for all SSB proteins - non-specific binding of a single-stranded DNA strand, as well as, discovered much later, RNA binding. SSB proteins play an essential role in processes closely related to ssDNA. They play an important role in DNA replication, recombination and repair. They are responsible for interactions with single-stranded DNA, prevent the formation of secondary structures and protect against the degrading effects of nucleases.

The discovery of SSB proteins dates back to the mid-60s of the twentieth century. The first proteins detected are the SSB protein phage T4 and *E. coli.* At that time, their strong properties associated with interaction with ssDNA and protein elution in the ssDNA-cellulose resin in the presence of a high salt concentration - 2 M NaCl were discovered. Attention was also drawn to the very high selectivity of this protein to single-stranded DNA. Confirmation of the fundamental role of SSB proteins in the processes associated with ssDNA is the fact that they occur in all living organisms, as well as in viruses.

The binding of the SSB protein to ssDNA consists in packing aromatic amino acid residues between bases in the oligonucleotide chain. In addition, positively charged amino acid residues interact with the phosphate the phosphate skeleton in the ssDNA moleculein the ssDNA molecule.

Despite the fact that the NeqSSB protein belongs to the SSB protein family, it differs in its characteristics from classic SSB proteins, hence it is referred to as the *Neq*SSB-like protein. This protein comes from the hyperthermophilic archaeon *Nanoarchaeum equitans*, which parasitizes in the craenarchaeon *Ignicoccus hospitalis.* The optimal growth conditions of this microorganism require strictly anaerobic conditions and a temperature of 90 °C. Interestingly, *Nanoarchaeum equitans* has the smallest known genome consisting of 490,885 base pairs. Unlike most known organisms with reduced genomes, it has a full set of enzymes involved in DNA replication, repair, and recombination, including the SSB protein.

The *Neq*SSB protein, like other proteins in this family, has a natural ability to bind DNA. It consists of 243 amino acid residues, and in its structure it has one OB domain (Fig. 1). It exhibits biological activity as a monomer, similar to some viral SSB proteins. Studies indicate its unusual ability for other SSB proteins to bind all forms of DNA (ssDNA, dsDNA) and mRNA without structural preferences. In addition, the protein is characterized by high thermostability. The half-life while maintaining biological activity is 5 minutes at 100°C, while the melting point is 100,2°C.

The purpose of the invention is to create a new Pwo-NeqSSB polymerase, which will be applicable to the duplication of specific sequences of the SARS CoV-2 virus.

The subject of the invention is Pwo-NeqSSB polymerase with SEQ. ID 1.

The subject of the invention is also the method of cloning Pwo-NeqSSB polymerase with SEQ.ID 1, by which DNA of the insert intended for cloning is obtained, which consists in carrying out two independent PCR reactions:
- in the first amplification reaction, a product with a nucleotide sequence corresponding to the sequence of the gene encoding the Pwo DNA polymerase is obtained with an additional linker sequence and complementary to the 11 initial nucleotides of the NeqSSB protein at the C-terminus;
- the second product contains the nucleotide sequence of the gene encoding the NeqSSB protein binding DNA to additional nucleotides characteristic of the linker and 11 additional nucleotides complementary to the final nucleotide sequence of Pwo polymerase at the N-terminus;
- the PCR templateis isolated genomic DNA,
- the resulting products are separated in the two above reactions in agarose gel with ethidine bromide and isolated from the gel.

A method where the products of two PCR reactions serve as inserts in the Gibson reaction, where:
- plasmid pET30EKLIC digestion
- in order to linearize the plasmid pET30EKLIC, it is digested with the enzyme BamHI and NdeI (NEB), which cut in two places leaving uncomlementary ends of DNA,
- the vector DNA digestion reaction is carried out for 2 hours at 37 °C with the addition of an appropriate buffer,
- the digested plasmid undergoes electrophoretic separation and isolates itself;
- Gene assembly reaction
- the Gibson reaction is carried out in a thermocycler at 50°C for 60 minutes, where the mixture contains buffer, nucleotides, enzymes, sterile water, insert I, Insert II, vector,
- after the reaction, the mixture is added to the freshly prepared competent cells of *E. coli* TOP10,
- the resulting mixture is incubated in ice for 40 minutes, after this incubation time, a thermal shock is carried out consisting in placing the cell mixture for 60 s in a thermoblock with a temperature of 42 ° C, and then 2 minutes of incubation in ice, after thermal shock the cells are subjected to 60 min incubation in 37°C with the addition of 600 ml LB, after this time the cells spin (10 min, 1800 rpm), 500 ml of filtrate was discarded, the sediment was suspended in the remaining amount of supernatant and was spread evenly over the plates with LA substrate with the addition of kanamycin, the plates were incubated for approximately 16 hours at 37 ° C.

Method where, in order to obtain the Pwo-Neq SSB fusion protein, E. coli BL RIL cells are transformed using the recombinant plasmid DNA pET30-Pwo-NeqSSB and the production of the desired fusion proteins is carried out, cultures with the addition of kanamycin and chloramphenicol are carried out for 16 hours at 37 °C, pre-inoculum is inoculated into fresh medium and after the cultures reach OD600 = 0,5, IPTG is added to the final concentration of 0,1 mM; after induction, cultures are carried out for another 5 hours, then spin (10 minutes, 5000 rpm) and are purified by metal-affinity chromatography; the results of protein production are analysed by polyacrylamide gel electrophoresis under denaturing conditions.

The subject of the invention is also an isolated recombinant plasmid, which includes a fragment of the nucleotide sequence of proteins encoding Pwo-NeqSSB polymerase from 5076 to 8168 nt from the plasmid pET30EKLIC with SEQ. ID. 5.

Isolated plasmid pET30-Pwo-NeqSSB with SEQ sequence. ID.5.

The subject of the invention are also primers for cloning Pwo-NeqSSB polymerase with sequences SEQ.ID. 6.

The subject of the invention is Pwo-NeqSSB polymerase with SEQ. ID 1 to be used for the replication of specific SARS CoV-2 virus sequences.

### Description of drawings (figures) and sequences:

**Fig. 1** - presents the quaternary structure of the DNA polymerase Pwo, which consists of the exonuclease domain (blue colour), the N-terminal domain (purple colour) and three subdomains: palm(orange colour), fingers (green colour), thumb (red colour).
**Fig. 2** - schematic depiction of the basic domains present in the NeqSSB protein
**Fig. 3** - presents the Gibson cloning method pattern.
**Fig. 4** - presents the electrophoretic separation of products after PCR reaction.
   A - separation of products after the first PCR reaction allowing to obtain a product of 762 bpsize.
   B - separation of products after the second PCR reaction allowing to obtain a product of 2372 bp size. M - HyperLadder II DNA marker, 1-4 expected PCR product.
**Fig. 5** - presents the electrophoretic separation of the plasmid pD454-SR DNA after the digestion reaction. M - DNA marker SM0311 (ThermoScientific), 1 - pET30EKLIC not digested, 2 - pET30EKLIC after digestion reaction
**Fig. 6** - presents the electrophoretic separation showing the results of purification of Pwo-Neq DNA polymerase on a column with a His-Trap resin
   *M - Molecular weight markers (14.4* - *116 kDa) with the size of standard proteins: 116; 66,2; 45; 35; 25; 18,4; 14.4 kDa;*
   1 - *E. coli BL RIL*/*pET30-Pwo-NeqSSB cell lysate after sonication and denaturation of host proteins*
   *2* - *Fraction after washing with buffer B (200 ml)*
   *3* - *Fraction after elution with buffer C (15 ml)*
   *4* - *Fraction after the second elution with buffer C (15 ml)*

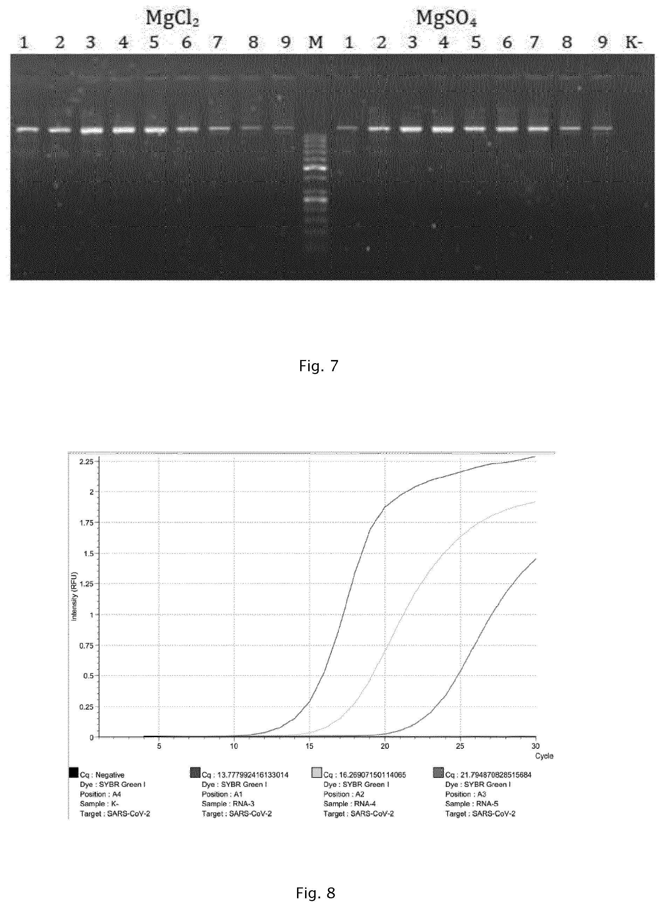

**Fig. 7** - presents the electrophoretic separation of products after PCR reaction with the addition of different concentrations of magnesium ions. 1 - 0.5 ul Mg2+ 50 mM, 2 - 0.75 ul Mg2+ 50 mM, 3 - 1 ul Mg2+ 50 mM, 4 - 1.25 ul Mg2+ 50 mM, 5 - 1.5 ul Mg2+ 50 mM, 6 - 1.75 ul Mg2+ 50 mM, 7 - 2 ul Mg2+ 50 mM, 8 - 2.25 ul Mg2+ 50 mM, 9 - 2.5 ul Mg2+ 50 mM, K- - without magnesium.
**Fig. 8** - presents a graph of the fluorescence dependence of the SybrGreen dye during the RT qPCR reaction using Pwo-Neq polymerase in the SARS-CoV-2 virus identification reaction on the tempate of isolated viral RNA.

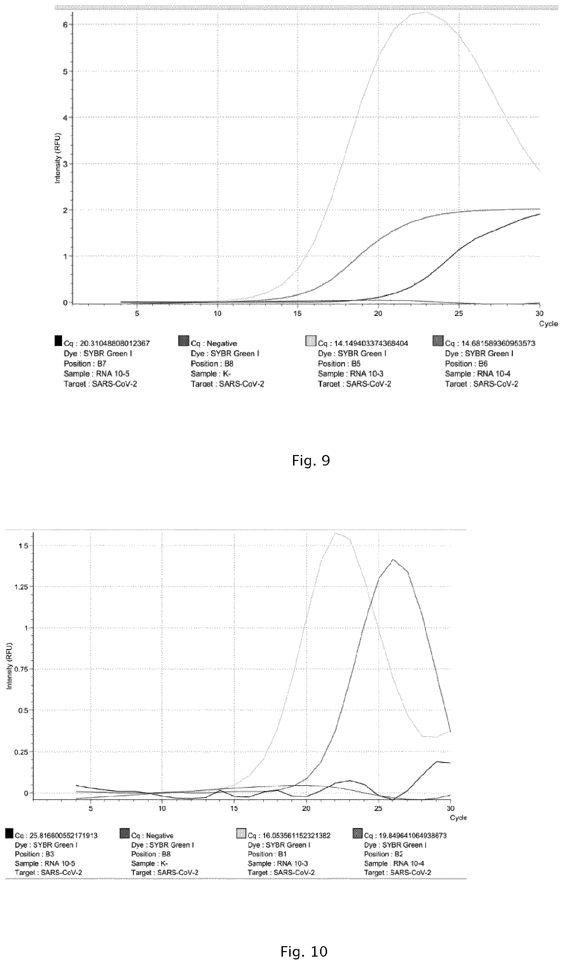

**Fig. 9** - presents a graph of the fluorescence dependence of the SybrGreen dye during the RT qPCR reaction with the use of Pwo-Neq polymerase in the SARS-CoV-2 virus identification reaction directly from the swab.

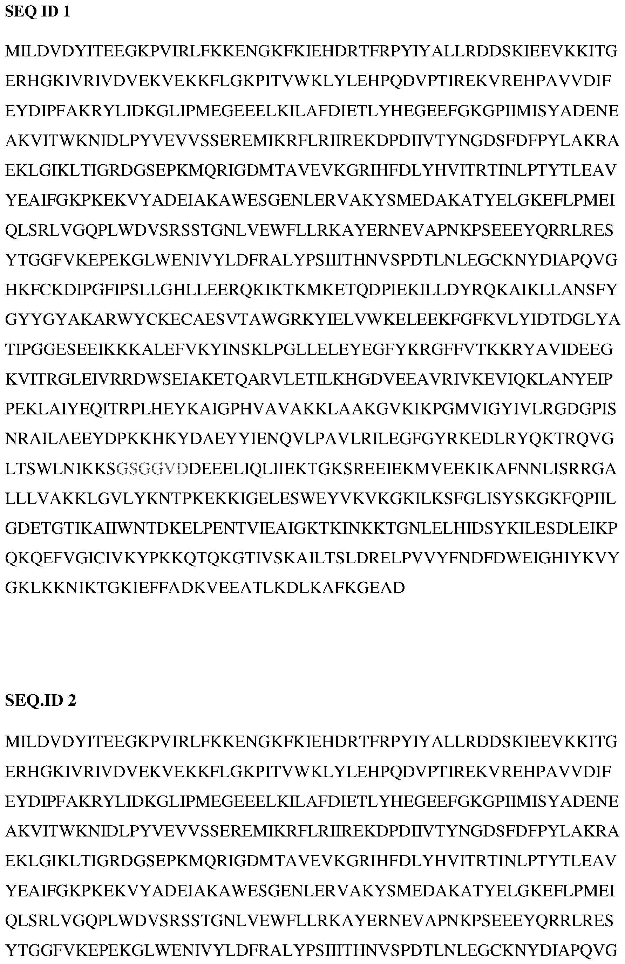

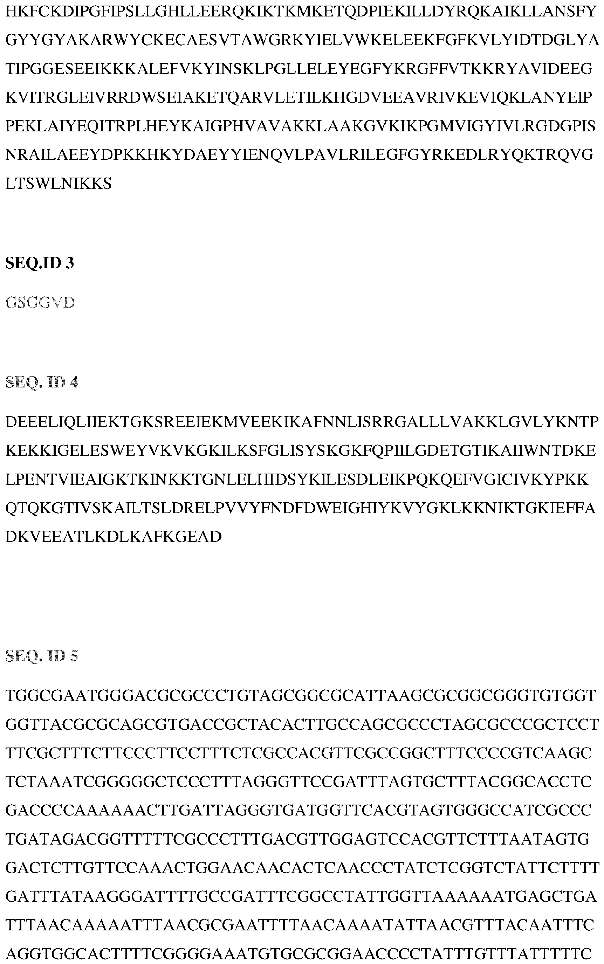

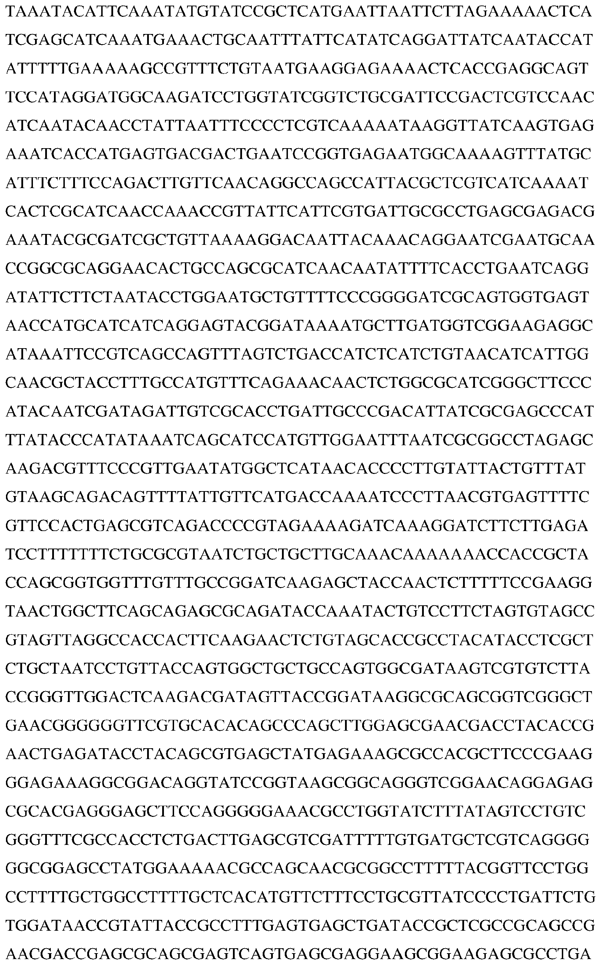

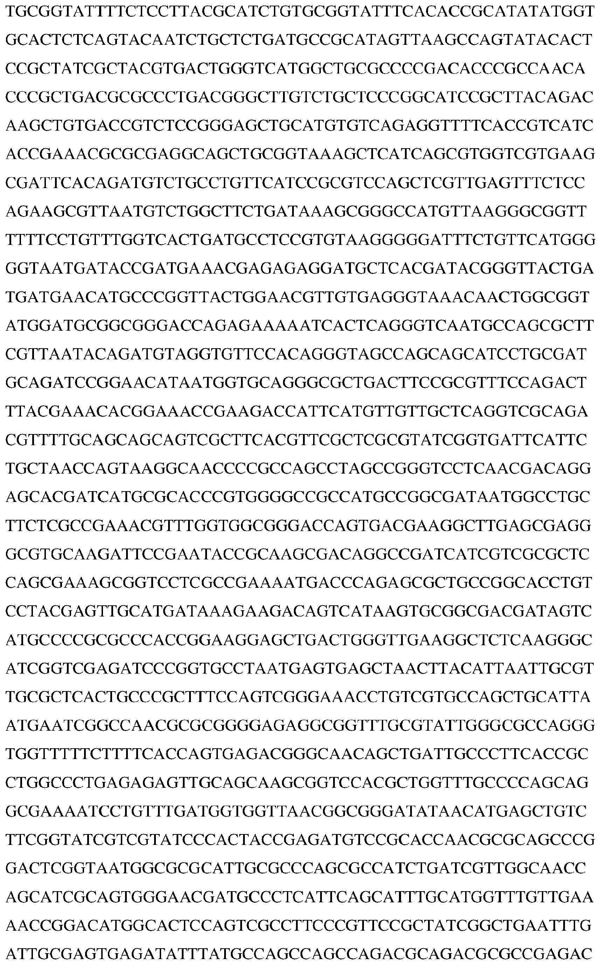

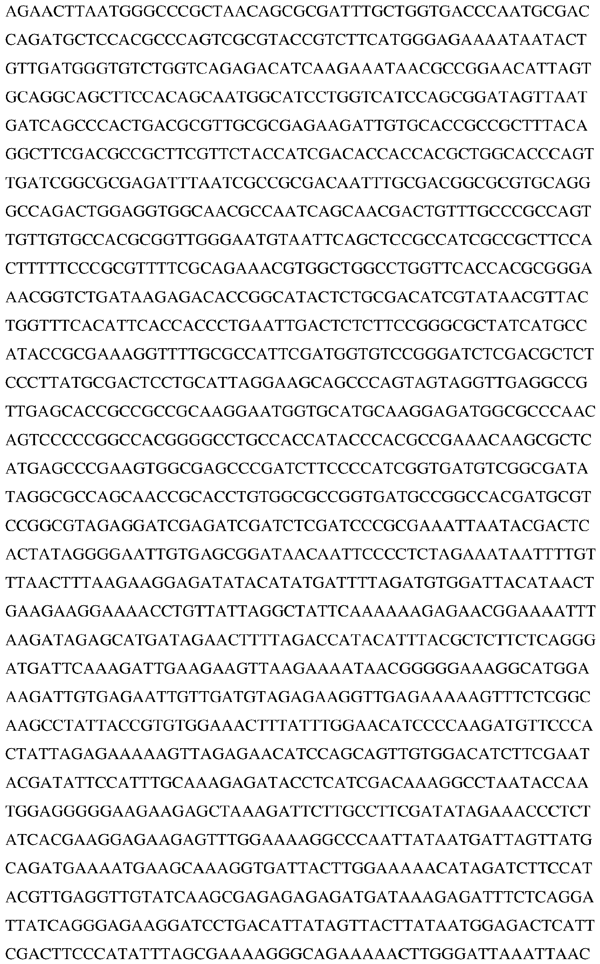

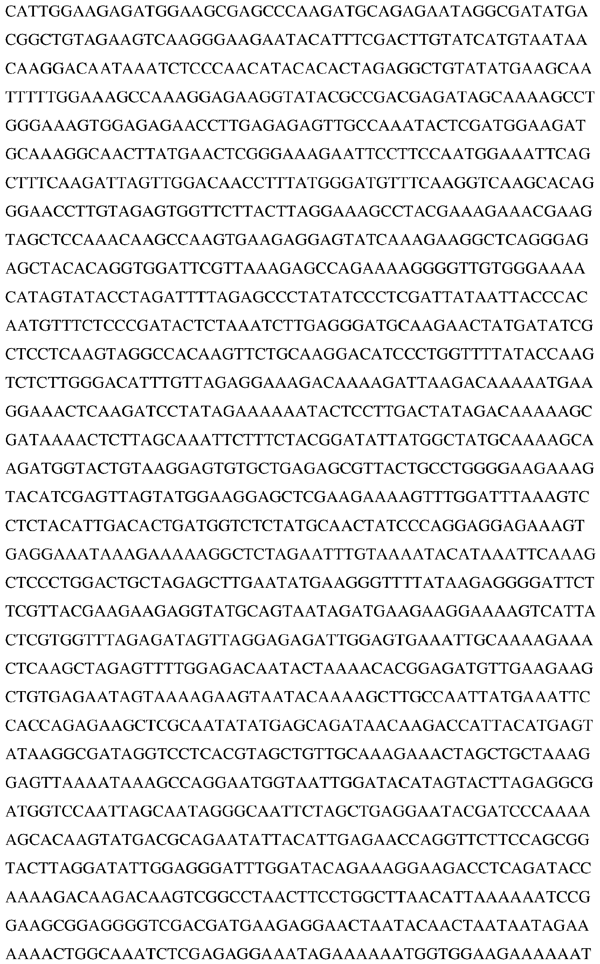

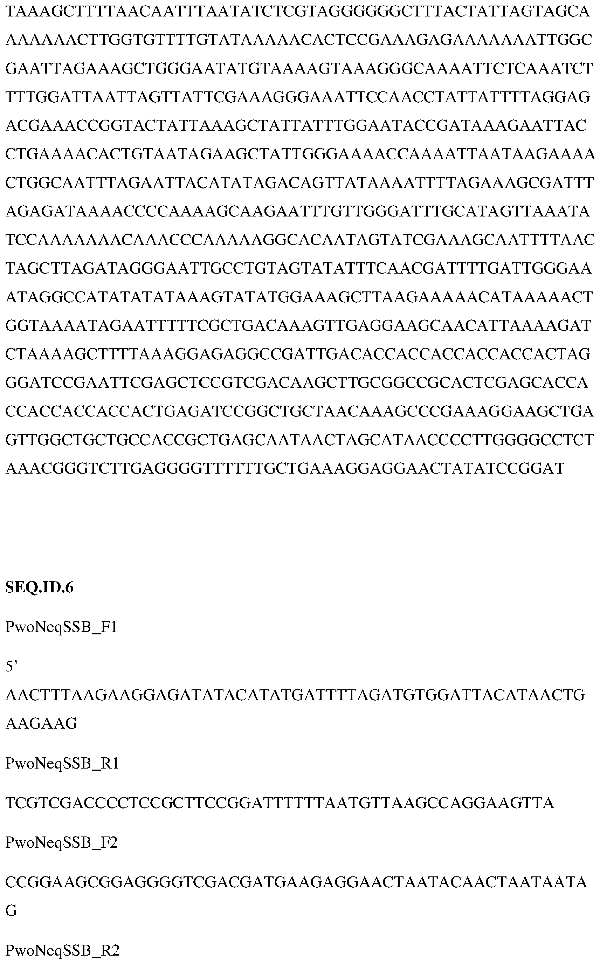

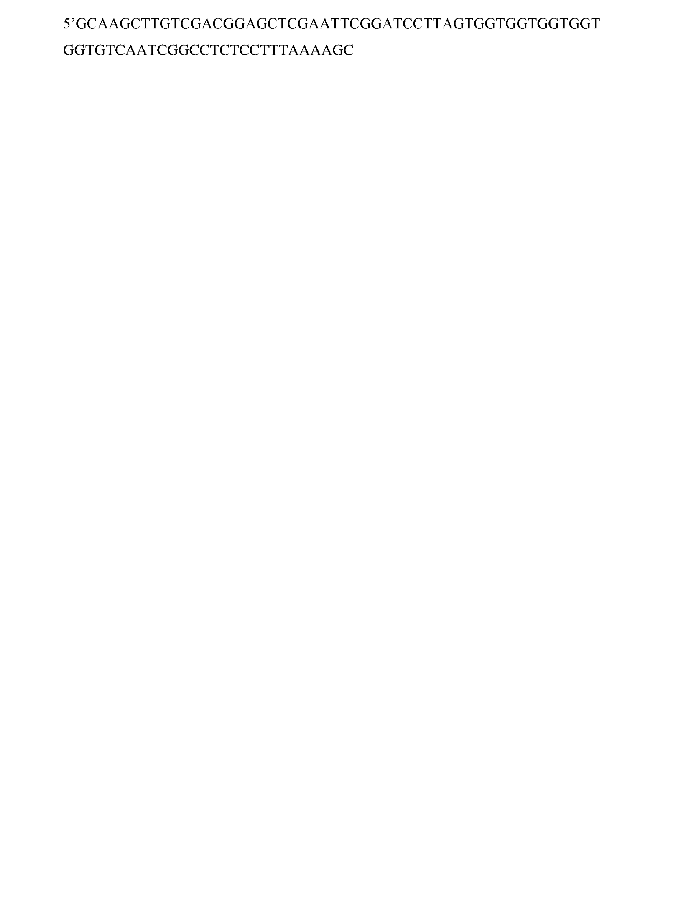

**Fig. 10** - presents a graph of the dependence of fluorescence of the SybrGreen dye during the RT qPCR reaction using Pwo-Neq polymerase in the SARS-CoV-2 virus identification reaction directly from saliva in the breaking buffer.

**SEQ. ID 1** - presents the amino acid sequence of the Pwo-NeqSSB fusion polymerase construct, where: The main core of the polymerase is the Pwo polymerase, which at its C-terminus, using a six-amino acid linker (GSGGVD), will be combined with the NeqSSB protein (isolated from *Nanoarchaeum equitans).* The presence of a linker provides the fusion protein with some flexibility and relatively free arrangement in relation to polymerase, which is aimed at preventing possible steric hindrance.
**SEQ.ID.2** - presents the amino acid sequence of Pwo polymerase
**SEQ.ID 3** - presents the amino acid sequence of the linker
**SEQ.ID. 4** - presents the amino acid sequence of the NeqSSB protein
**SEQ. ID. 5** - presents the sequence of a plasmid with a gene encoding the Pwo-NeqSSB protein
**SEQ.ID. 6** - presents the sequences of primers

The invention is illustrated by the following examples of execution, which do not constitute its limitations.

### Example 1:

### I. Polymerase coding gene, expression vector, expression system

### a) Gene encoding Pwo-NeqSSB polymerase

The amino acid sequence of Pwo-NeqSSB polymerase was extended by the sequence of the histidine domain necessary for effective purification of polymerase by metal-affinity chromatography. The 6xHis domain was attached to the C-terminus of the polymerase. The nucleotide sequence of polymerase is presented on SEQ ID 1.

### b) Expression vector

When choosing a vector for enzyme expression, the following were guided by the possibility:
- antibiotic selection of clones
- cloning and its multiplication in bacterial cells
- the presence of a promoter enabling an easy way to control and induce expression

The pET30EKLIC vector carrying the kanamycin resistance gene, the bacterial Ori replication and the T7 lactose promoter sequence allowing for induction of expression by IPTG were selected. The vector has recognition sites for the BamHI and NdeI restriction enzymes, which cut plasmid DNA in two places giving two non-complementary sticky ends necessary at the cloning stage.

### c) Expressive system

For the expression of thermostable fusion polymerase Pwo-NeqSSB, a prokaryotic system - *E. coli* was chosen, which is the most commonly used system for overproduction of proteins on both laboratory and industrial scales. IP-Free strains available by Promega have been selected: *Escherichia coli* BL21(DE3)pLysS.

### d) Designing SEQ.ID 6 primers

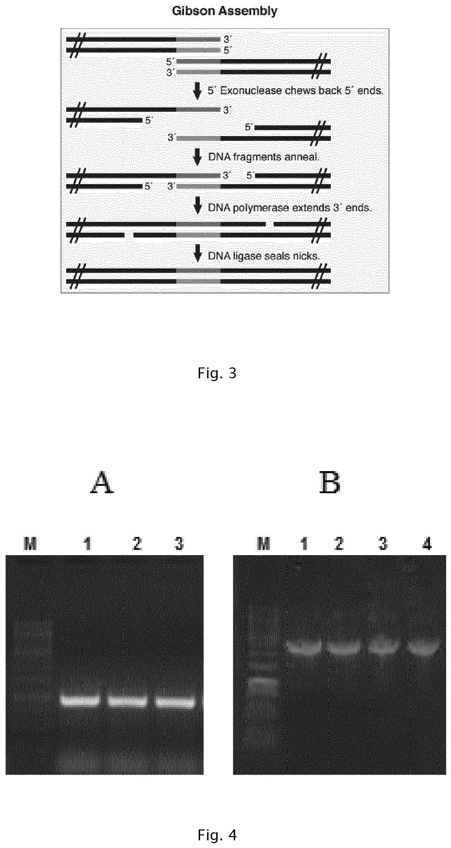

Phusion polymerase consists of two proteins that are encoded by two independent genes. This forces the use of the cloning method, which will allow the cloning of several DNA fragments at the same time. It was decided to use the Gibson method, which in one reaction allows to generate sticky ends (exonuclease 5'→3'), lengthen DNA ends (DNA polymerases) and covalent connection of two ENDS OF DNA (DNA ligase) of several fragments at the same time. The OverLap set from A&A Biotechnology was used. The cloning diagram is shown in Figure 3.

### Example 2:

### II. Pwo-Neq SSB SSB polymerase cloning

### a) Amplification of products intended for cloning

Obtaining the DNA of the insert intended for cloning consisted in carrying out two independent PCR reactions. The first amplification reaction produced a product with a nucleotide sequence corresponding to the DNA polymerase sequence with an additional linker sequence and complementary to the 11 initial nucleotides of the NeqSSB protein at the C-terminus. The second product contained a nucleotide sequence of a DNA-binding protein with additional nucleotides characteristic of the linker and 11 additional nucleotides complementary to the final nucleotide sequence of Pwo polymerase at the N-terminus.

PCR template was isolated genomic DNA. The obtained products in the two above reactions were separated in agarose gel with ethidium bromide and subjected to isolation from the gel using the Gel-Out Concentrator (A&A Biotechnology) set. The result of the obtained products after the PCR reaction is shown in Figure 4.

The products of these two PCR reactions served as inserts in the Gibson reaction using the OverLap Assembly mix (A&A Biotechnology).

### b) Plasmid pET30EKLIC etching

In order to linearize the plasmid pET30EKLIC DNA, it was digested with the enzyme BamHI and NdeI (NEB), which cuts in two places leaving uncomlementary ends of DNA. The reaction of vector DNA digestion was carried out for 2 hours at a temperature of 37 ° C with the addition of an appropriate buffer recommended by the manufacturer.

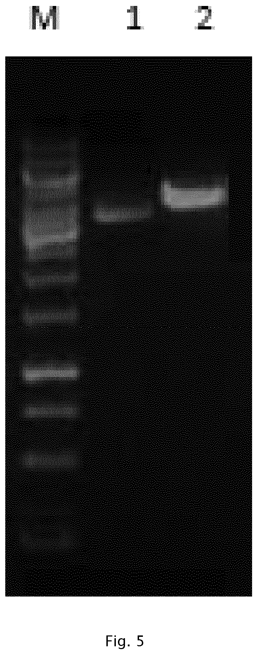

The digested plasmid was subjected to electrophoretic separation and isolated with a Gel-Out Concentrator (A&A Biotechnology) set. The result of digestion is shown in Figure 5.

### c) Gene assembly reaction

Gibson's reaction using the OverLap Assembly mix was carried out in a thermal cycler at 50°C for 60 minutes. The composition of the DNA template is presented below:

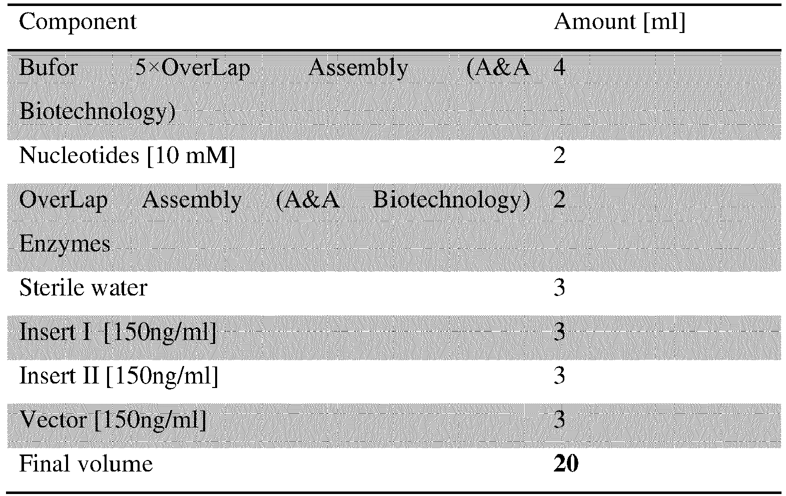

After the reaction, the mixture was added to freshly prepared cells competent *E. coli* TOP10.

### d) Transformation of competent cells

The mixture after the Gibson reaction was added to 100 ml of competent cells of *E. coli* TOP10. The resulting mixture was incubated in ice for 40 minutes. After this incubation time, a thermal shock was carried out, consisting in placing a mixture of cells for 60 seconds in a thermoblock with a temperature of 42 ° C, and then 2 minutes of incubation in ice. After thermal shock, the cells were incubated for 60 minutes at 37° C with the addition of 600 ml of LB. After this time, the cells were centrifuged (10 minutes, 1800 rpm), 500 ml of filtrate was discarded, the sediment was suspended in the remaining amount of supernatant and spread evenly over the plates with LA substrate with the addition of kanamycin. The plates were incubated for approximately 16 hours at 37°C.

### Example 3:

### III. Expression and purification of Pwo-Neq SSB polymerase

In order to obtain the Pwo-NeqSSB fusion protein, E. coli BL RIL cells were transformed using the DNA of the recombinant plasmid pET30-Pwo-NeqSSB and the production of the desired fusion proteins was carried out. Cultures with the addition of kanamycin and chloramphenicol were carried out for 16 hours at 37°C, pre-inoculum was inoculated into fresh medium and after the cultures reached OD600 = 0.5, IPTG was added to the final concentration of 0.1 mM. After induction, the cultures were carried out for another 5 hours, after which they were centrifuged (10 min, 5000 rpm) and subjected to purification by the metal-affinity chromatography method. The results of protein production were analyzed using polyacrylamide gel electrophoresis under denaturing conditions (SDS-PAGE) (Figure 5). The molecular mass of the protein calculated using the Expasy computer programme was 118.2 kDa:

### Number of amino acids: 1023

### Molecular mass: 118219.00

### Theoretical pI: 8.46

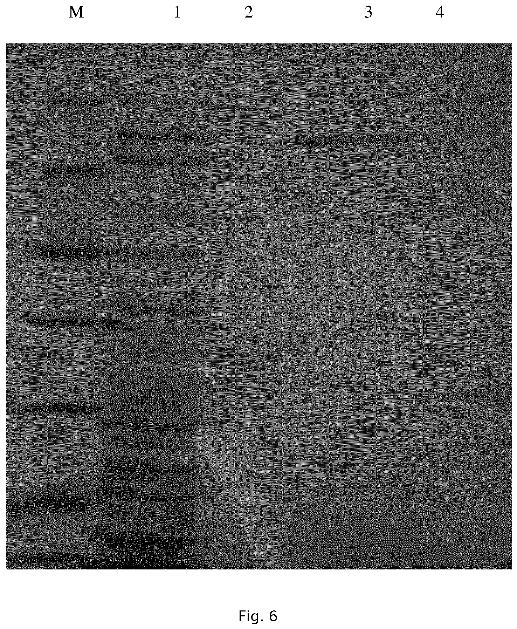

The results of electrophoretic separations showing the degree of purification and the concentration of proteins after each purification step are shown in Figure 6.

### Example 4:

### IV. Activity and application of Pwo-Neq SSB polymerase

1) The use of polymerase in a classic PCR reaction with detection in agarose gel - target fragment of plasmid DNA pUC19 with a size of 1200 bpin different concentrations of magnesium ions, Fig. 7.
2) The use of polymerase in the real-time PCR reaction - identification of the S gene using various RNA template: total RNA of the SARS-CoV-2 virus, directly from the swab infected with the virus as well as saliva with the SARS-CoV-2 virus. (Fig. 8-10).

### Literature:

[1] Vieille C, Burdette DS, Zeikus JG. Thermozymes. Biotechnol Annu Rev. 1996;2:1-83.
[2] Hamilton SC, Farchaus JW, Davis MC. DNA polymerases as engines for biotechnology. Biotechniques. 2001;31(2):370-6, 378-80, 382-3.
[3] Kim SW, Kim DU, Kim JK, Kang LW, Cho HS. Crystal structure of Pfu, the high fidelity DNA polymerase from Pyrococcus furiosus. Int J Biol Macromol. 2008;42 (4): 356-61.
[4] Takagi M, Nishioka M, Kakihara H, Kitabayashi M, Inoue H, Kawakami B, Oka M, Imanaka T. Characterization of DNA polymerase from Pyrococcus sp. strain KOD1 and its application to PCR. Appl Environ Microbiol. 1997;63(11):4504-10.
[5] Olszewski M, Balsewicz J, Nowak M, Maciejewska N, Cyranka-Czaja A, Zalewska-Piatek B, et al. Characterization of a Single-Stranded DNA-Binding-Like Protein from Nanoarchaeum equitans - Nucleic Acid Binding Protein with Broad Substrate Specificity. PLoS One. 2015; 10:e0126563.
[6] DNA Modifying enzymes.; Avaliable from: https:/www.neb.com/~/media/Catalog/All-Products/0AA961B29E444AFBEDD5C4A904C76E6/Datacards or Manusals/ManualE2611.pdf

## Claims

1. Pwo-NeqSSB polymerase o SEQ. ID 1.

2. A method of cloning Pwo-NeqSSB polymerase with SEQ.ID 1 **characterized in that** the DNA of the insert intended for cloning is obtained, which consists in carrying out two independent PCR reactions:
- during the first amplification reaction a product with a nucleotide sequence corresponding to the sequence of the gene encoding the Pwo DNA polymerase with an additional linker sequence and complementary to the 11 initial nucleotides of the NeqSSB protein at the C-terminus is obtained;
- the second product contains the nucleotide sequence of the gene encoding the NeqSSB protein binding DNA to additional nucleotides characteristic of the linker as well as 11 additional nucleotides complementary to the final nucleotide sequence of Pwo polymerase at the N-terminus;
- the PCR template is isolated genomic DNA,
- the resulting products are separated in the two above reactions in agarose gel with ethidine bromide and isolated from the gel.

3. The method according to claim 2, **characterized in that** the products of two PCR reactions serve as inserts in the Gibson reaction, where:
- plasmid pET30EKLIC digestion
- in order to linearize the plasmid pET30EKLIC, it is digested with the enzyme BamHI and NdeI (NEB), which cut in two places leaving uncomlementary ends of DNA,
- the vector DNA digestion reaction is carried out for 2 h at 37 degrees Celsius with the addition of an appropriate buffer,
- the digested plasmid undergoes electrophoretic separation and isolates itself;
- Gene assembly reaction
- the Gibson reaction is carried out in a thermocycler at 50°C for 60 minutes, where the mixture contains buffer, nucleotides, enzymes, sterile water, insert I, Insert II, vector,
- after the reaction, the mixture is added to the freshly prepared competent cells of *E. coli* TOP10,
- the resulting mixture is incubated in ice for 40 minutes, after this incubation time, a thermal shock is carried out consisting in placing the cell mixture for 60 s in a thermoblock with a temperature of 42 ° C, and then 2 minutes of incubation in ice, after thermal shock the cells are subjected to 60 min incubation in 37°C with the addition of 600 ml LB, after this time the cells spin (10 min, 1800 rpm), 500 ml of filtrate was discarded, the sediment was suspended in the remaining amount of supernatant and spread evenly over the plates with LA substrate with the addition of kanamycin, the plates were incubated for approximately 16 h at 37 ° C.

4. The method according to claim 3 **characterized in that** in order to obtain the Pwo-Neq SSB fusion protein, E. coli BL RIL cells are transformed using the recombinant plasmid DNA pET30-Pwo-NeqSSB and the production of the desired fusion proteins is carried out, cultures with the addition of kanamycin and chloramphenicol are carried out for 16 h at 37°C, pre-inoculum is inoculated into fresh medium and after the cultures reach OD600 = 0,5, IPTG is added to the final concentration of 0,1 mM; after induction, cultures are carried out for another 5 h, then spin (10 minutes, 5000 rpm) and are purified by metal-affinity chromatography; the results of protein production are analysed by polyacrylamide protein electrophoresis under denaturing conditions.

5. Isolated recombinant plasmid **characterized in that** it includes a fragment of the nucleotide sequence of proteins encoding Pwo-NeqSSB polymerase from 5076 to 8168 from the plasmid pET30EKLIC o SEQ. ID. 5.

6. Isolated plasmid pET30-Pwo-NeqSSB with SEQ.ID.5.

7. Pwo-NeqSSB polymerase cloning primers with sequences SEQ.ID. 6.

8. Pwo-NeqSSB polymerase o SEQ. ID 1 to be used for the replication of specific SARS CoV-2 virus sequences.
